(19) Europäisches Patentamt
European Patent Office
Office européen des brevets

(11) Publication number: **0 275 952 B1**

# (12) EUROPEAN PATENT SPECIFICATION

(45) Date of publication of patent specification: **24.07.91**

(51) Int. Cl.5: **C07C 319/06**, C07C 323/20

(21) Application number: **88100607.6**

(22) Date of filing: **18.01.88**

## (54) Process for preparing 2,6-ditertiarybutyl-4-mercaptophenol and 4,4'-isopropylidenedithiobis-(2,6-di-tertiarybutylphenol).

(30) Priority: **20.01.87 US 5510**

(43) Date of publication of application:
**27.07.88 Bulletin 88/30**

(45) Publication of the grant of the patent:
**24.07.91 Bulletin 91/30**

(84) Designated Contracting States:
**AT BE CH DE ES FR GB GR IT LI LU NL SE**

(56) References cited:
**EP-A- 0 200 212**
**GB-A- 1 199 871**
**US-A- 3 275 694**
**US-A- 3 479 407**
**US-A- 3 576 883**
**US-A- 3 952 064**

**HOUBEN-WEYL, "Methoden der organischen Chemie", vol. IX, 1955, Georg Thieme-Verlag Stuttgart; pp. 23-29**

(73) Proprietor: **MERRELL DOW PHARMACEUTICALS INC.**
**2110 East Galbraith Road**
**Cincinnati Ohio 45215-6300(US)**

(72) Inventor: **Laskovics, F. Marck**
**814 Coolidge Drive**
**Midland, MI 48640(US)**
Inventor: **Bargeron, Kim G.**
**3618 Westbrier Terrace**
**Midland, MI 48640(US)**

(74) Representative: **Sgarbi, Renato et al**
**GRUPPO LEPETIT S.p.A. Patent and Trademark Department 34, Via Roberto Lepetit**
**I-21040 Gerenzano (Varese)(IT)**

**EP 0 275 952 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a novel, improved process of making 2,6-di-tertiarybutyl-4-mercaptophenol by a cobalt-molybdenum catalyzed reduction of bis(3,5-di-tertiarybutyl-4-hydroxyphenol)polysulfide in the presence of hydrogen gas. This mercaptophenol is an intermediate in the synthesis of 4,4'-isopropylidenedithio-bis-(2,6-di-tertiarybutylphenol) which has been disclosed in U.S. Patent 3,576,883 as an effective pharmaceutical agent for the reduction of serum cholesterol. This invention further relates to a novel, improved process of making 4,4'isopropylidenedithio-bis-(2,6-di-tertiarybutylphenol) by further reacting the 2,6-di-tertiarybutyl-4-mercaptophenol thus formed with acetone in the presence of acid.

U.S. Patent 3,479,407 teaches the preparation of a mixture of bis(3,5-di-tertiarybutyl-4-hydroxyphenol)-polysulfide, comprising principally the disulfide, by a process of sulfurization of 2,6-di-tertiarybutylphenol (DTBP) with sulfur monochloride in the presence of an iodine catalyst. The polysulfide has been shown to be reduced to 2,6-di-tertiarybutyl-4-mercaptophenol by a process comprising a Zn/HCl reduction as disclosed in U.S. Patents 3,952,064 and 3,479,407 and in Japanese Patent Application 73-28425. Condensation of the resulting mercaptophenol in the presence of acetone under acidic conditions results in the formation of 4,4'-isopropylidenedithio-bis-(2,6-di-tertiarybutylphenol), as described in U.S. Patent 3,576,883. This reaction sequence is presented below.

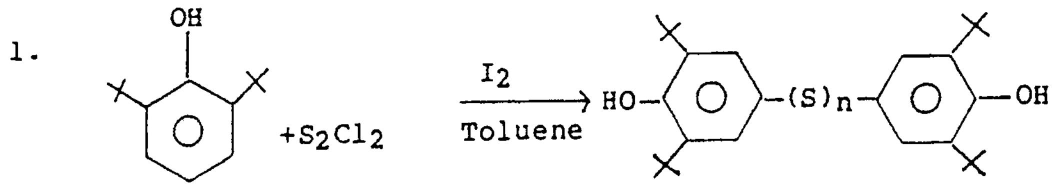


(I) DTBP

(II) Polysulfide

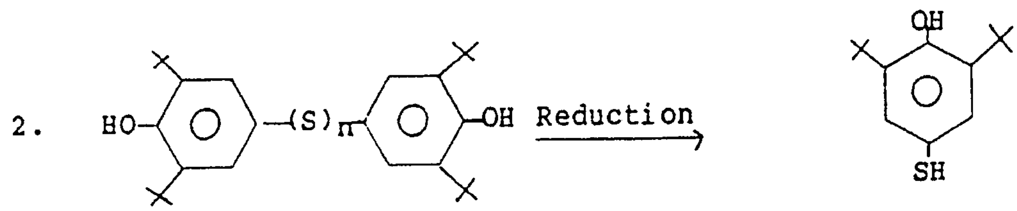


(II) Polysulfide

(III) Mercaptophenol

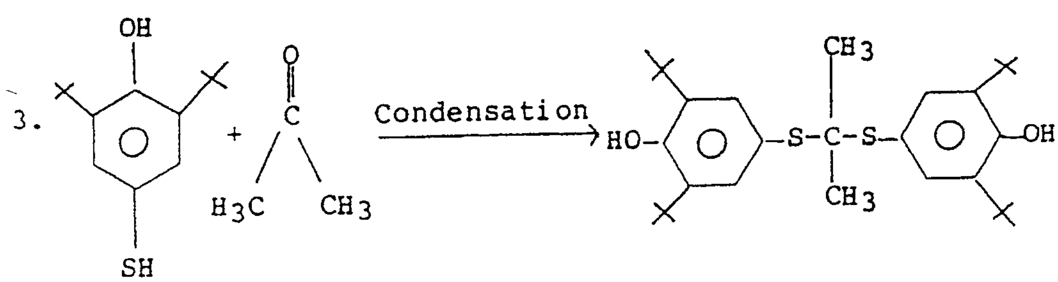


(III) Mercaptophenol

(IV) 4,4'-isopropylidenedithio-bis-(2,6-di-tertiarybutylphenol)

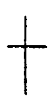 = tertiarybutyl group

n = 2,3,4,...; principal product is the disulfide

The following terms are used herein as follows:

"DTBP" refers to 2,6-di-tertiarybutylphenol (I).

"Polysulfide" and "Bis(3,5-di-tertiarybutyl-4-hydroxyphenyl)polysulfide" both are used to refer to one or more species of bis(3,5-di-tertiarybutyl-4-hydroxyphenyl)polysulfide (II) including the di-, tri-, tetra-, and other higher order sulfides, and including single species as well as mixtures thereof. Typically, the Polysulfide is a mixture of two or more species with the disulfide present in amounts greater than other species.

"Mercaptophenol" refers to 2,6-di-tertiarybutyl-4-mercaptophenol (III).

"Catalytic amount" refers to that amount of a Cobalt-Molybdenum preparation which is sufficient to catalyze the reduction of Polysulfide to Mercaptophenol in the presence of Hydrogen gas. This amount may vary under particular conditions of use whereby other experimental parameters are varied including concentration of Polysulfide, absolute amount of Polysulfide, hydrogen gas pressure, temperature, residence time on the trickle-bed reactor column, and surface area of the catalyst.

"g" and "ml" refer to grams and millilitres, respectively.

The novel improvement in the process of making the Mercaptophenol and 4,4′-isopropylidenedithio-bis-(2,6-di-tertiarybutylphenol) comprises a cobalt-molybdenum catalyzed hydrogenation of the Polysulfide and represents a significant improvement over the Zn/HCl reduction described for this reaction in U.S. Patent 3,479,407. The Zn/HCl reduction requires a lengthy and tedious batch reduction with subsequent crystallization in order to isolate the Mercaptophenol for the condensation step to form 4,4′-isopropylidenedithio-bis-(2,6-di-tertiarybutylphenol).When the improved process is carried out utilizing a fixed-bed reactor, it provides a streamlined, continuous-flow method of making the Mercaptophenol. The reaction mixture effluent can be utilized directly in the condensation step to form 4,4′-isopropylidenedithio-bis-(2,6′-di-tertiarybutylphenol) without prior isolation of the Mercaptophenol. Thus, the present improved process allows Polysulfide, which is introduced into a trickle-bed reactor containing a fixed-bed, cobalt-molybdenum catalyst in the presence of hydrogen gas to be converted to the Mercaptophenol which is present in the reaction-mixture effluent. The effluent can then be reacted in a continuous or a batch-wise manner with acetone under acidic conditions to form 4,4′-isopropylidenedithio-bis-(2,6-di-tertiarybutylphenol) without prior isolation of the Mercaptophenol.

The trickle-bed reactor in which the hydrogenation takes place can comprise a jacketed pipe configured as a vertical column in which sufficient catalyst, hydrogen pressure and temperature can be maintained to support the reaction. The reactor is charged with a catalytic amount of cobalt-molybdenum catalyst. The preferred cobalt-molybdenum catalyst comprises about 5% cobalt oxide and about 16% molybdenum oxide on an inert support material. Alumina is preferred as the support material. The amount of surface area provided by a particular catalyst preparation can be chosen so as to provide optimum hydrogenation results. The feed solution or liquid can enter at the top of the column by means of a pump. A particular gas such as hydrogen can be introduced into the reactor and regulated by means of a pressure regulator. The feed solution mixes with the gas and cascades through the catalyst bed with the feed solution maintaining contact with both the catalyst and the hydrogen gas throughout the reactor. The stream then exits into the liquid level control tank in which the liquid is allowed to collect in order to provide a liquid seal to maintain the gas pressure. The effluent collected in the liquid level control tank is allowed to exit such that the level of liquid in the tank is maintained.

The Polysulfide can be prepared by sulfur monochloride sulfurization of DTBP preferably as described in U.S. Patent 3,479,407. The Polysulfide can be dissolved in any solvent in which it is soluble and which is non-reactive under the conditions set forth below. Toluene is preferred as the solvent. The trickle-bed reactor can be prepared as described above and the cobalt-molybdenum catalyst can be sulfided by treating with $H_2S$. Hydrogen gas can then be introduced at a minimum pressure of about 600 psig (42.3 Kg $cm^{-2}$) to a maximum pressure comprising the upper limit maintainable by the system. A hydrogen pressure of about 600 psig (42.3 Kg $cm^{-2}$) to about 2000 psig (141 Kg $cm^{-2}$) is preferred with about 750 psig (52.9 Kg $cm^{-2}$) being most preferred. The temperature can be maintained at about 80 °C to about 120 °C with about 100 °C being preferred. The in- and the out-flow rates of the solvent can be adjusted so that the liquid level in the control tank is maintained and so that the residence time of the solvent on the column is about 3 minutes to about 20 minutes with about 9 minutes to about 12 minutes being preferred. The Polysulfide can be introduced onto the column and the resulting effluent from the trickle-bed reactor will contain the Mercaptophenol. This effluent can then be reacted further with acetone under acidic conditions to form 4,4′-isopropylidenedithio-bis-(2,6-ditertiarybutylphenol).

The reaction conditions, in particular the amount and surface area of the cobalt-molybdenum catalyst, the concentration and absolute amount of the Polysulfide used, and the residence time on the column as well as the column temperature and hydrogen pressure, can be adjusted to provide the highest yield of Mercaptophenol in the effluent. The optimum parameters can be easily determined by one skilled in the art by simple experimentation.

The following example illustrates the cobalt-molybdenum catalyzed hydrogenation of Polysulfide to make the Mercaptophenol utilizing a fixed-bed catalyst containing about 5% by weight of CoO and about 16% by weight of $MoO_3$ on an inert support material. In addition, the Mercaptophenol thus formed is converted to 4,4′-isopropylidenedithio-bis-(2,6-di-tertiarybutylphenol) by treating the reaction mixture with acetone under acidic conditions.

EXPERIMENTAL RESULTS

A trickle-bed reactor (1-inch; 2.4cm; diameter) was loaded with 67 g (80 ml) of 1/4 inch (0.635 cm) ceramic packing, followed by 244 g (346 ml) of Co-Mo catalyst consisting of 5.0% by weight of CoO and 16.2% by weight of $MoO_3$ on approximately 1/8 inch x 1/16 inch (0.327 cm x 0.163 cm) elliptical ribbed alumina (American Cyanamid Trilobe HDS-20-1.6), followed by 43 g (50 ml) of 1/4 inch (0.635 cm) ceramic packing. The cobalt-molybdenum catalyst was sulfided by purging the reactor with nitrogen at room temperature, warming to 150° C, pressurizing to 100 psig (7.05 Kg $cm^{-2}$) nitrogen, and treating with 30 g $H_2S$ over an hour period. Toluene was used as the liquid seal. The column was maintained at 100° C and 750 psig (52.9 kg $cm^{-2}$) hydrogen gas.

A toluene solution containing Polysulfide produced by the sulfurization of DTBP, as described in U.S. Patent 3,479,407, was applied to the trickle-bed reactor with a feed flow rate of 464 ml/hour. This flow rate provided a residence time of the substrate on the catalyst of about 9 minutes. The Polysulfide was almost completely reduced to the corresponding Mercaptophenol as indicated by High Performance Liquid Chromatography (HPLC) of the reactor effluent.

The Mercaptophenol was further reacted to form 4,4′-isopropylidenedithio-bis-(2,6-di-tertiarybutylphenol) by reacting 500.7 g of effluent from the trickle-bed reactor (containing an estimated 680 millimoles of Mercaptophenol) with 65.6 g (1.26 moles) of acetone, followed by 7.7 g (0.21 moles) of HCl (gas). After reacting at 20° C for 3 hours, the reaction mixture was quenched with 100 ml of water. After stirring for 15 minutes, the layers were permitted to separate, and the bottom water layer was removed and discarded. The organic solvent was removed under reduced pressure and the residue was recrystallized three times from 90% ethanol/water yielding 4,4′-isopropylidenedithio-bis-(2,6-di-tertiarybutylphenol) with a melting point of 124.5° C to 128° C.

## Claims

1. In a process for making 2,6-di-tertiarybutyl-4-mercaptophenol by reduction of a bis(3,5-di-tertiarybutyl-4-hydroxyphenol)polysulfide the improvement which comprises reducing the bis(3,5-di-tertiarybutyl-4-hydroxyphenol)polysulfide with hydrogen gas in the presence of a catalytic amount of a cobalt-molybdenum catalyst.

2. The process of Claim 1 wherein the catalyst comprises about 5% by weight of cobalt oxide and about 16% by weight of molybdenum oxide on a solid inert support.

3. The process of Claim 1 wherein the reduction is carried out at a temperature of about 80° C to about 120° C.

4. The process of Claim 1 wherein the hydrogen gas is maintained at a pressure of about 600 psig (42.3 Kg $cm^{-2}$) to about 2000 psig (141 Kg $cm^{-2}$).

5. The process of Claim 1 wherein the reduction is carried out in a continuous flow manner by passing a solution of bis(3,5-di-tertiarybutyl-4-hydroxyphenol)polysulfide in an inert solvent through a fixed bed of the catalyst.

6. The process of Claim 5 wherein the 2,6-di-tertiarybutyl-4-mercaptophenol is continuously withdrawn from the effluent of the fixed catalyst bed.

7. In a process for making 4,4′-isopropylidenedithio-bis-(2,6-di-tertiarybutylphenol) by reduction of a bis (3,5-di-tertiarybutyl-4-hydroxyphenol)polysulfide and by further reacting the reduction product with acetone under acidic conditions, the improvement which comprises reducing the bis(3,5-di-tertiarybutyl-4-hydroxyphenol)polysulfide with hydrogen gas in the presence of a catalytic amount of cobalt-molybdenum catalyst.

8. The process of claim 7 wherein the catalyst comprises about 5% by weight of cobalt oxide and about 16% by weight of molybdenum oxide on a solid inert support.

9. The process of Claim 7 wherein the reduction is carried out at a temperature of about 80° C to about 120° C.

10. The process of Claim 7 wherein the hydrogen gas is maintained at a pressure of about 600 psig (42.3 Kg $cm^{-2}$) to about 2000 psig (141 Kg $cm^{-2}$).

11. The process of Claim 7 wherein the reduction is carried out in a continuous flow manner by passing a solution of bis(3,5-di-tertiarybutyl-4-hydroxyphenol)polysulfide in an inert solvent through a fixed bed of the catalyst.

12. The process of Claim 11 wherein the 2,6-di-tertiarybutyl-4-mercaptophenol is continuously withdrawn from the effluent of the fixed catalyst bed and then further reacted with acetone and acid.

13. The process of Claim 11 wherein the resulting reaction mixture is reacted directly with acetone in the presence of acid.

**Revendications**

1. Dans un procédé pour la fabrication du 2,6-di-tertio-butyl-4-mercaptophénol par réduction d'un polysulfure de bis (3,5-di-tertiobutyl-4-hydroxyphényle),le perfectionnement qui comprend la réduction du polysulfure de bis (3,5-di-tertiobutyl-4-hydroxyphényle) par le gaz hydrogène en présence d'une quantité catalytique d'un catalyseur cobalt-molybdène.

2. Le procédé selon la revendication 1, dans lequel le catalyseur comprend environ 5 % en poids d'oxyde de cobalt et environ 16 % en poids d'oxyde de molybdène sur un support inerte solide.

3. Le procédé selon la revendication 1, dans lequel la réduction est mise en oeuvre à une température d'environ 80 à environ 120 °C.

4. Le procédé selon la revendication 1, dans lequel le gaz d'hydrogène est maintenu sous une pression d'environ 42,3 à environ 141 bar (600 à 2 000 psig).

5. Le procédé selon la revendication 1, dans lequel la réduction est mise en oeuvre en courant continu par passage du polysulfure de bis (3,5-di-tertiobutyl-4-hydroxyphényle) dans un solvant inerte à travers un lit fixe du catalyseur.

6. Le procédé selon la revendication 5, dans lequel le 2,6-di-tertiobutyl-4-mercaptophénol est retiré en continu de l'effluent du lit fixe du catalyseur.

7. Dans un procédé de fabrication du 4,4'-isopropylidènedithio-bis-(2,6-di-tertiobutylphénol) par réduction d'un polysulfure de bis(3,5-di-tertiobutyl-4-hydroxyphényle) et par réaction ultérieure du produit de réduction avec l'acétone en conditions acides, le perfectionnement qui comprend la réduction du polysulfure de bis(3,5-di-tertiobutyl-4-hydroxyphényle) par le gaz hydrogène en présence d'une quantité catalytique d'un catalyseur cobalt-molybdène.

8. Le procédé selon la revendication 7, dans lequel le catalyseur comprend environ 5 % en poids d'oxyde de cobalt et environ 16 % en poids d'oxyde de molybdène sur un support inerte solide.

9. Le procédé selon la revendication 7, dans lequel la réduction est mise en oeuvre à une température d'environ 80 à environ 120 °C.

10. Le procédé selon la revendication 7, dans lequel le gaz d'hydrogène est maintenu sous une pression d'environ 600 psig. (42.3 Kg $cm^{-2}$) a environ 2000 psig. (141 Kg $cm^{-2}$).

11. Le procédé selon la revendication 7, dans lequel la réduction est mise en oeuvre en courant continu par passage du polysulfure de bis (3,5-di-tertiobutyl-4-hydroxyphényle) dans un solvant inerte à travers un lit fixe du catalyseur.

12. Le procédé selon la revendication 11, dans lequel le 2,6-di-tertiobutyl-4-mercaptophénol est retiré en continu de l'effluent du lit fixe du catalyseur et après fait réagir avec l'acétone et un acide.

13. Le procédé selon la revendication 11, dans lequel le mélange réactionnel résultant est directement mis à réagir avec l'acetone en présence d'un acide.

**Patentansprüche**

1. Verbesserung eines Verfahrens zur Herstellung von 2,6-Di-tert.-butyl-4-mercaptophenol durch Reduktion eines Bis-(3,5-di-tert.-butyl-4-hydroxyphenol)polysulfids, umfassend die Reduktion von Bis(3,5-di-tert.-butyl-4-hydroxyphenol)polysulfid mit Wasserstoffgas in Gegenwart einer katalytischen Menge eines Cobalt-Molybdän-Katalysators.

2. Verfahren nach Anspruch 1, wobei der Katalysator etwa 5 Gew.-% Cobaltoxid und etwa 16 Gew.-% Molybdänoxid auf einem festen inerten Träger umfaßt.

3. Verfahren nach Anspruch 1, wobei die Reduktion bei einer Temperatur von etwa 80 bis etwa 120 °C durchgeführt wird.

4. Verfahren nach Anspruch 1, wobei das Wasserstoffgas bei einem Druck von etwa 600 psig (42,3 kg $cm^{-2}$) bis etwa 2000 psig (141 kg $cm^{-2}$) gehalten wird.

5. Verfahren nach Anspruch 1, wobei die Reduktion mit kontinuierlichem Durchfluß durchgeführt wird, indem eine Lösung von Bis(3,5-di-tert.-butyl-4-hydroxyphenol)polysulfid in einem inerten Lösungsmittel durch ein Festbett des Katalysators geleitet wird.

6. Verfahren nach Anspruch 5, wobei das 2,6-Di-tert.-butyl-4-mercaptophenol aus dem Abstrom des Festbettkatalysators kontinuierlich entnommen wird.

7. Verbesserung eines Verfahrens zur Herstellung von 4,4'-Isopropylidendithio-bis-(2,6-di-tert.-butylphenol) durch Reduktion eines Bis(3,5-di-tert.-butyl-4-hydroxyphenol)-polysulfids und durch weitere Umsetzung des Reaktionsprodukts mit Aceton unter sauren Bedingungen, die die Reduktion von Bis(3,5-di-tert.-butyl-4-hydroxyphenol)polysulfid mit Wasserstoffgas in Gegenwart einer katalytischen Menge des Cobalt-Molybdän-Katalysators umfaßt.

8. Verfahren nach Anspruch 7, wobei der Katalysator etwa 5 Gew.-% Cobaltoxid und etwa 16 Gew.-% Molybdänoxid auf einem festen inerten Träger umfaßt.

9. Verfahren nach Anspruch 7, wobei die Reduktion bei einer Temperatur von etwa 80 bis etwa 120 °C durchgeführt wird.

10. Verfahren nach Anspruch 7, wobei das Wasserstoffgas bei einem Druck von etwa 600 psig (42,3 kg $cm^{-2}$) bis etwa 2000 psig (141 kg $cm^{-2}$) gehalten wird.

11. Verfahren nach Anspruch 7, wobei die Reduktion unter kontinuierlichem Durchfluß durchgeführt wird, indem eine Lösung von Bis(3,5-di-tert.-butyl-4-hydroxyphenol)polysulfid in einem inerten Lösungmittel durch ein Festbett des Katalysators geleitet wird.

12. Verfahren nach Anspruch 11, wobei das 2,6-Di-tert.-butyl-4-mercaptophenol aus dem Abstrom des Festbettkatalysators kontinuierlich entfernt und sodann mit Aceton und Säure weiter umgesetzt wird.

13. Verfahren nach Anspruch 11, wobei das entstehende Reaktionsgemisch direkt mit Aceton in Gegenwart einer Säure umgesetzt wird.